(19) Europäisches Patentamt
European Patent Office
Office européen des brevets

(11) **EP 4 549 471 A1**

(12) **EUROPEAN PATENT APPLICATION**
published in accordance with Art. 153(4) EPC

(43) Date of publication:
07.05.2025 Bulletin 2025/19

(21) Application number: 23830917.3

(22) Date of filing: 25.05.2023

(51) International Patent Classification (IPC):
*C08B 1/00* (2006.01)      *C08J 3/12* (2006.01)
*A61Q 19/00* (2006.01)      *A61K 8/73* (2006.01)

(52) Cooperative Patent Classification (CPC):
A61K 8/73; A61Q 19/00; C08B 1/00; C08B 16/00;
C08J 3/12

(86) International application number:
PCT/JP2023/019567

(87) International publication number:
WO 2024/004466 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 01.07.2022 JP 2022107237

(71) Applicant: Futamura Kagaku Kabushiki Kaisha
Nakamura-ku
Nagoya-shi
Aichi 450-0002 (JP)

(72) Inventors:
• IWATA, Ippei
  Ogaki-shi Gifu 503-0932 (JP)
• SUZUKI, Tsubasa
  Ogaki-shi Gifu 503-0932 (JP)
• YAMAZAKI, Asuka
  Ogaki-shi Gifu 503-0932 (JP)

(74) Representative: TBK
Bavariaring 4-6
80336 München (DE)

(54) **UNMODIFIED CELLULOSE BEADS AND METHOD FOR MANUFACTURING SAME**

(57)   [SUMMARY]

[PROBLEM] To provide unmodified cellulose beads which achieve both textures and soft-focus properties suitable for use in cosmetics, and a production method therefor.

[SOLVING MEANS] Unmodified cellulose beads for mixing into a cosmetic, having an average particle diameter (D50) of 1 to 20 $\mu$m, an average particle diameter (D10) of 4.0 $\mu$m or less, an average particle diameter (D20) of 6.0 $\mu$m or less, and an average particle diameter (D90) of 30.0 $\mu$m or less, wherein a standard deviation (SD) of average particle size divided by the average particle diameter (D50) (SD/D50) is 0.4 to 1.5.

EP 4 549 471 A1

FIG. 1

Flowchart showing process steps:

MERCERIZATION STEP (S1)
- CELLULOSE → (ALKALI METAL HYDROXIDE) → HEATING/STIRRING → MERCERIZED CELLULOSE

DEPOLYMERIZATION STEP (S2)
- MERCERIZED CELLULOSE → AGING → RAW CELLULOSE

DEFIBRATION STEP (S3)
- RAW CELLULOSE → (ALKALI METAL HYDROXIDE) → (SOLVENT) → DEFIBRATION → CELLULOSE FINE FIBERS

NEUTRALIZATION STEP (S4)
- CELLULOSE FINE FIBERS → (ACID) → NEUTRALIZATION/WASHING/RE-DEFIBRATION → TYPE II UNMODIFIED CELLULOSE FINE FIBERS

DRYING STEP (S5)
- SPRAY-DRYING

COLLECTION STEP (S6)
- COLLECTION → UNMODIFIED CELLULOSE BEADS

## Description

FIELD

The present invention relates to unmodified cellulose beads and a production method therefor.

BACKGROUND

In recent years, the United Nations has set international goals for sustainable development referred to as the Sustainable Development Goals (SDGs), and one of these goals is to reduce the amount of plastic used, as an environmental issue. Efforts are being made to solve climate change by reducing the amounts of petroleum-based plastics used, thereby reducing GHG emissions.

For example, microplastics (beads) are sometimes used in cosmetics such as foundations to improve the mixability with other ingredients and the spread and feel during use. However, microplastics (beads) have been raised as a problem, and in particular, as one of the causes of worsening pollution of the marine environment, and efforts are being made to reduce the amount of microplastics generated and to recover them.

For these reasons, substitutes for microplastics (beads) are increasingly being in the market. However, the use of alternative materials has not progressed for fine resin raw materials such as microplastics (beads) having a particle size of 100 μm or less, and it is known that the supply is low as compared to the increasing demand.

Furthermore, cellulose, a biodegradable natural material, has been attracting attention as an alternative raw material to microplastics. Generally, cellulose is processed using the viscose process, which chemically modifies and dissolves the cellulose. However, since this process uses chemicals such as organic solvents which have a high environmental impact, there are concerns regarding the environment and product safety. Though not a dissolution-based system, it is possible to form molded bodies from cellulose fine fibers such as cellulose nanofibers and cellulose microfibers, which can be processed into beads or films, and are expected to be an alternative raw material to microplastics.

Examples of production methods for cellulose fine fibers include a method in which cellulose is oxidized in water using a catalyst and the resulting oxidized cellulose is defibrated to obtain a cellulose nanofiber dispersion (refer to Patent Literature 1); a method in which, in the carboxymethylation of cellulose, carboxymethylation is carried out in a mixed solvent of water and an organic solvent, and the resulting carboxymethylated cellulose is defibrated to obtain a highly transparent carboxymethylated cellulose nanofiber dispersion (refer to Patent Literature 2); and a production method in which an anion-modified cellulose nanofiber salt is desalted by carrying out a cation exchange reaction using a cation exchange resin to obtain anion-modified cellulose nanofibers (refer to Patent Literature 3).

The cellulose fine fibers obtained by these production methods are type I cellulose fine fibers having a small fiber diameter, and are chemically modified cellulose that is obtained by chemical defibration and mechanical (physical) defibration. Specifically, since defibration is performed using chemicals such as organic solvents, not only does the process require a chemical removal step, complicating the process, but there are also issues such as chemicals remaining in the product making it unusable in cosmetics and the like, limiting applications and raising concerns regarding the safety and environmental impact of the chemicals used.

In contrast, since unmodified cellulose, which does not use chemical defibration, does not use chemicals such as organic solvents during defibration, and does not have the issues such as chemicals remaining in the cellulose fine fibers after defibration, the unmodified cellulose is expected to be usable in a wide range of applications such as cosmetics. In particular, unmodified cellulose beads obtained from unmodified cellulose can serve as a substitute for microplastics used in cosmetics.

Generally, it is necessary that the microplastics used in cosmetics have suitable textures such as slipperiness. The textures of cosmetics containing microbeads are thought to be affected by the fineness and uniformity of the microbead particles. While cosmetics are also required to have soft-focus properties such as dullness removal, it has conventionally been difficult to achieve both textures and soft-focus properties in cosmetics containing conventional microplastics. Thus, the present inventors have repeatedly studied and improved unmodified cellulose beads obtained from unmodified cellulose as an alternative to microplastics used in cosmetics. As a result, unmodified cellulose beads which achieve both textures and soft-focus properties suitable for use in cosmetics, as well as a production method therefor have been invented.

[CITATION LIST]

[PATENT LITERATURE]

[PTL 1] Japanese Unexamined Patent Publication (Kokai) No. 2008-001728
[PTL 2] Japanese Unexamined Patent Publication (Kokai) No. 2019-99758
[PTL 3] WO 2019/059079

SUMMARY

[TECHNICAL PROBLEM]

The present invention has been conceived in light of the points described above, and provides unmodified cellulose beads which achieve both textures and soft-focus properties suitable for use in cosmetics, as well as a production method therefor.

[SOLUTION TO PROBLEM]

Specifically, the invention of claim 1 relates to unmodified cellulose beads for mixing into a cosmetic, having an average particle diameter (D50) of 1 to 20 $\mu$m, an average particle diameter (D10) of 4.0 $\mu$m or less, an average particle diameter (D20) of 6.0 $\mu$m or less, and an average particle diameter (D90) of 30.0 $\mu$m or less, wherein a standard deviation (SD) of average particle size divided by the average particle diameter (D50) (SD/D50) is 0.4 to 1.5.
The invention of claim 2 relates to the unmodified cellulose beads according to claim 1, which are produced from a raw material pulp without being subjected to a defibration step by chemical defibration.
The invention of claim 3 relates to the unmodified cellulose beads according to claim 2, having a Hunter Whiteness (W value) of 85 or more and a Hunter Yellowness (YI value) of 20 or less.
The invention of claim 4 relates to the unmodified cellulose beads according to claim 1, wherein a dispersion in which 0.05% by weight of the unmodified cellulose beads are dispersed in 50% trimethylsiloxysilicate and 50% cyclopentasiloxane has a haze value of 50% or more.
The invention of claim 5 relates to a production method for the unmodified cellulose beads according to claim 1, the method comprising a mercerization step of mercerizing a cellulose to obtain mercerized cellulose, a depolymerization step for reducing a degree of polymerization of the mercerized cellulose to 760 or less, a defibration step in which a raw cellulose obtained via the mercerization step and the depolymerization step is defibrated by adding an alkali metal hydroxide to the raw cellulose so that the total concentration of the alkali metal hydroxide is 2.5 to 17.5%, to obtain cellulose fine fibers, a neutralization step of neutralizing the cellulose fine fibers with an acid, and a drying step of spray-drying the neutralized cellulose fine fibers at an air pressure of 0.025 to 0.6 MPa.

[ADVANTAGEOUS EFFECTS OF INVENTION]

According to the unmodified cellulose beads of the invention of claim 1, since there are provided unmodified cellulose beads for mixing into a cosmetic, having an average particle diameter (D50) of 1 to 20 $\mu$m, an average particle diameter (D10) of 4.0 $\mu$m or less, an average particle diameter (D20) of 6.0 $\mu$m or less, and an average particle diameter (D90) of 30.0 $\mu$m or less, wherein a standard deviation (SD) of average particle size divided by the average particle diameter (D50) (SD/D50) is 0.4 to 1.5, it is possible to achieve both textures and soft-focus properties suitable for use in cosmetics.
According to the unmodified cellulose beads of the invention of claim 2, since the unmodified cellulose beads in the invention of claim 1 are produced from a raw material pulp without being subjected to a defibration step by chemical defibration, the safety thereof in cosmetic applications is improved.
According to the unmodified cellulose beads of the invention of claim 3, since the unmodified cellulose beads in the invention of claim 2 have a Hunter Whiteness (W value) of 85 or more and a Hunter Yellowness (YI value) of 20 or less, high purity cellulose beads can be obtained.
According to the unmodified cellulose beads of the invention of claim 4, since a dispersion in which 0.05% by weight of the unmodified cellulose beads in the invention of claim 1 are dispersed in 50% trimethylsiloxysilicate and 50% cyclopentasiloxane has a haze value of 50% or more, excellent soft-focus properties can be achieved.
According to the production method of the invention of claim 5 for unmodified cellulose beads, since there is provided a production method for the unmodified cellulose beads according to claim 1, the method comprising a mercerization step of mercerizing a cellulose to obtain mercerized cellulose, a depolymerization step for reducing a degree of polymerization of the mercerized cellulose to 760 or less, a defibration step in which a raw cellulose obtained via the depolymerization step and the depolymerization step is defibrated by adding an alkali metal hydroxide to the raw cellulose so that the total concentration of the alkali metal hydroxide is 2.5 to 17.5%, to obtain cellulose fine fibers, a neutralization step of neutralizing the cellulose fine fibers with an acid, and a drying step of spray-drying the neutralized cellulose fine fibers at an air pressure of 0.025 to 0.6 MPa, unmodified cellulose beads which achieve both textures and soft-focus properties suitable for use in cosmetics can be obtained.

BRIEF DESCRIPTION OF DRAWINGS

FIG. 1 is an operation flowchart illustrating a production method for unmodified cellulose beads according to an embodiment of the present invention.
FIG. 2 is a graph showing the particle size distributions of Trial Examples 1 to 4.
FIG. 3 is a graph showing the particle size distributions of Trial Examples 5 to 7 and Comparative Example 1.

DESCRIPTION OF EMBODIMENTS

The unmodified cellulose beads of the present invention are to be mixed with a cosmetic and are used as an alternative raw material to microplastics (beads). The cellulose beads are a molded body of cellulose fine fibers, and from the viewpoint of textures when added to cosmetics, type II cellulose fine fibers are preferably used, which are softer than type I cellulose fine fibers having a strong type I crystal structure. The unmodified cellulose beads are a molded body of type II cellulose fine fibers which are not chemically modified (unmodified), and from the viewpoint of safety in cosmetic applications, it is preferable that they be produced from raw a material pulp without being subjected to a defibration step by chemical defibration.

Pulp is a raw material primarily produced by pulverizing wood and removing impurities such as lignin to increase the purity of the cellulose components. Cotton linter pulp, which is produced by removing impurities from cotton to increase the purity of the cellulose components, can also be used. Furthermore, pulp is fibrous and highly reactive with chemicals, making it a preferable cellulose raw material. In addition to pulp, animal cellulose such as bacterial cellulose produced by microorganisms can also be used. Purified cellulose obtained by refining these raw materials can be used.

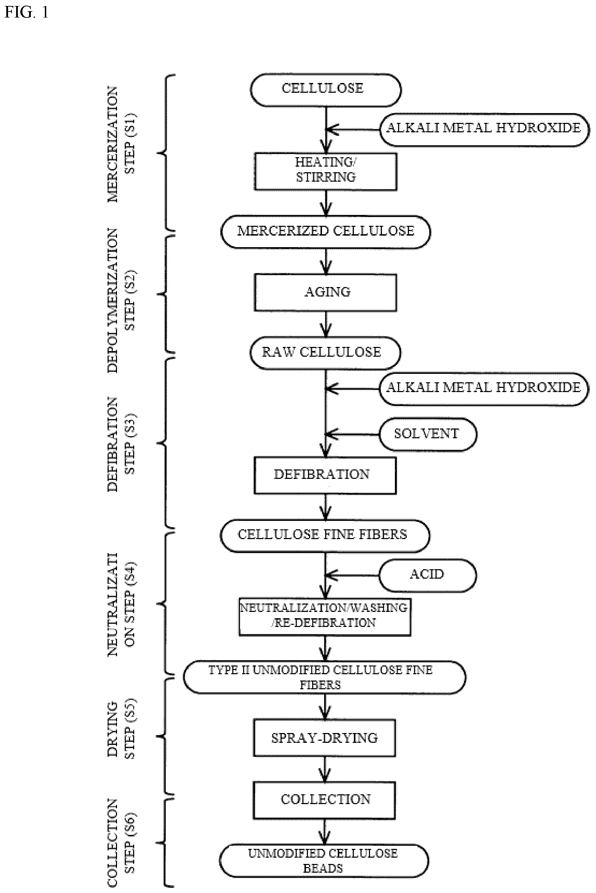

The unmodified cellulose beads of the present invention are obtained by a production method comprising a mercerization step (S1), a depolymerization step (S2), a defibration step (S3), a neutralization step (S4), and a drying step (S5), as shown in FIG. 1.

The mercerization step (S1) is a step in which cellulose is mercerized to obtain mercerized cellulose. In the mercerization step, the raw cellulose is added to an alkali metal hydroxide such as caustic soda (NaOH), and the mixture is heated and stirred as necessary to cause the cellulose fibers to swell. When the cellulose fibers are immersed in an alkali metal hydroxide, they are negatively charged and a Coulomb force is generated, which causes the individual fibers to repel each other and make them susceptible to defibration. Since the mercerized cellulose is susceptible to defibration as described above, it is possible to reduce the energy required in the subsequent defibration step (S3).

Examples of the alkali metal hydroxide used in the mercerization step include caustic soda (NaOH), lithium hydroxide, and potassium hydroxide. From the viewpoints of cost, safety, and environmental impact, caustic soda is preferably used.

After the mercerization step (S1), excess alkali metal hydroxide is removed as necessary. The solid content concentration is appropriately adjusted, and a depolymerization step (S2) is performed. The depolymerization step (S2) is a step for reducing the degree of polymerization of the mercerized cellulose obtained in the mercerization step (S1) to 760 or less. The mercerized cellulose having the adjusted solid content is appropriately pulverized and then aged by oxidative decomposition with oxygen in the air to reduce the degree of polymerization. In this case, the degree of polymerization is set to 760 or less. When the degree of polymerization of the mercerized cellulose is set to 760 or less, the transparency of the dispersion of the obtained cellulose fine fibers is secured. Furthermore, the lower the degree of polymerization of the mercerized cellulose, the easier it is to defibrate the cellulose fibers in the subsequent defibration step.

The aging of the mercerized cellulose in the depolymerization step (S2) is carried out at room temperature or under heating conditions. In order to accelerate the depolymerization rate, heating conditions which do not dry the raw material are preferably used. An aging accelerator, such as manganese(II) sulfate, which accelerates the aging reaction can be added.

By carrying out the mercerization step (S1) and the depolymerization step (S2), raw cellulose which can be defibrated into fine fibers is obtained. By mercerizing cellulose, the raw cellulose becomes type II cellulose having a type II crystal structure. Type II cellulose is said to be inferior to type I cellulose in terms of strength, etc., but since the cellulose fine fibers obtained by the present invention are intended to be used as a substitute for plastics in the field of cosmetics, etc., they do not require the same strength as type I cellulose, and thus, the decrease in strength is not a problem.

In the defibration step (S3), an alkali metal hydroxide and a solvent (ion-exchanged water) are added to the raw cellulose to adjust the total concentration to 2.5 to 17.5%, and defibration is performed. As described above, the alkali metal hydroxide used here can be caustic soda, lithium hydroxide, potassium hydroxide, etc., and from the viewpoints of cost and safety, caustic soda is preferably used. The raw cellulose is defibrated by mechanical (physical) defibration.

Mechanical (physical) defibration is performed by a known method using a homogenizer, water jet, etc. Since the raw cellulose is in a state where the fibers are swollen and susceptible to defibration by mercerization, and the degree of polymerization is reduced by the depolymerization step, defibration can be easily performed without applying high pressure, which is also advantageous in terms of equipment.

If the concentration of the alkali metal hydroxide is less than 2.5%, the swelling of the cellulose may be insufficient, and defibration may be difficult. If the concentration of the alkali metal hydroxide is greater than 17.5%, the salt concentration becomes high, whereby the cellulose fibers are more likely to aggregate, which may make defibration more difficult. If the alkali metal hydroxide is outside the range and defibration is insufficient, un-defibrated fibers remain in the resulting dispersion of cellulose fine fibers, resulting in low transparency and insufficient uniformity, which may cause fibrous substances to be mixed into the cellulose beads or the surface condition of the cellulose beads to become rough, resulting in a defective product.

Defibration may be performed in a plurality of steps. For example, by performing preliminary defibration using a mixer and then primary defibration using a homogenizer, cellulose fine fibers having uniform fiber diameters and small fiber diameters can be obtained. Preliminary defibration can avoid problems such as clogging of the defibration device with the raw cellulose. Preliminary defibration is performed by a known method using a mixer, refiner, etc. The defibration of cellulose fine fibers is sufficient if the average fiber diameter is defibrated to a nano-size to several hundred nano-size. When the average fiber diameter is approximately 2 to 800 nm, and more preferably 100 nm or less, the transparency of the dispersion of cellulose fine fibers is improved and the processing suitability of cellulose beads is improved.

The cellulose fine fibers obtained via the defibration step (S3) are neutralized with an acid in the neutralization step (S4). The cellulose fine fibers obtained via the defibration step are strongly alkaline and thus require neutralization. Examples of acids which can be used include sulfuric acid, hydrochloric acid, and lactic acid. The neutralized cellulose fine fibers are appropriately washed and re-defibrated to obtain type II unmodified cellulose fine fibers.

The drying step (S5) is a step in which the dispersion of cellulose fine fibers obtained via the neutralization step (S4) is spray-dried to form unmodified cellulose beads. The dispersion of cellulose fine fibers is dried and is caused to aggregate by spray drying, and formed into granules (beads). The conditions for spray drying include an air pressure of 0.025 to 0.6 MPa. If the air pressure for spray drying is insufficient, the cellulose fine fibers are not atomized, whereby the particle size becomes large and the physical properties and textures thereof deteriorate. Furthermore, if the air pressure is higher than 0.6 MPa, the air generator becomes excessively large, which is not practical.

In the method for the production of unmodified cellulose beads of the present invention, a collection step (S6) is carried out after the drying step (S5) as necessary. The collection step (S6) is a step of removing unnecessary particles from the cellulose beads formed via the drying step (S5) to recover cellulose beads of suitable particles. The collection means is not particularly limited as long as it is capable of recovering suitable particles, and collection can be carried out by known collection means such as a bag filter or a cyclone dust collector. With the unmodified cellulose beads obtained in this manner, the amount of chemicals having a high environmental impact can be reduced as compared to conventional cellulose beads.

The unmodified cellulose beads obtained by the production method of the present invention have an average particle size (D50) at 50% cumulative value, an average particle diameter (D10) at 10% cumulative value, an average particle diameter (D20) at 20% cumulative value, and an average particle diameter (D90) at 90% cumulative value, in the particle size distribution, and a standard deviation (SD) of average particle size divided by the average particle diameter (D50) (CV value: SD/D50) as specified below. Note that the average particle size (D50) is the median diameter, and the average particle sizes (D10), (D20), (D90) and the CV value (SD/D50) indicate the tendency of the particle size distribution, and in particular, the CV value (SD/D50) is an index indicating the uniformity of the particle size, and the smaller the value thereof, the more uniform the particle size.

In the particle size distribution, the average particle size (D50) at 50% cumulative value is 1 to 20 $\mu$m, and more preferably 2.5 to 10 $\mu$m. The average particle diameter (D10) at 10% cumulative value is 4.0 $\mu$m or less, and more preferably 3.0 $\mu$m or less. The average particle diameter (D20) at 20% cumulative value is 6.0 $\mu$m or less, and more preferably 4.5 $\mu$m or less. The average particle diameter (D90) at 90% cumulative value is 30.0 $\mu$m or less, and more preferably 20.0 $\mu$m or less. The CV value (SD/D50) is 0.4 to 1.5, and more preferably 0.5 to 1.0.

The unmodified cellulose beads of the present invention are particles having a median diameter (D50) of 1 to 20 $\mu$m and a D90 of 30.0 $\mu$m or less, contain a large number of fine particles such as those having a D10 of 4.0 $\mu$m or less and a D20 of 6.0 $\mu$m or less, and have a CV value (SD/D50) of 0.4 to 1.5, and thus, the particles as a whole have a relatively non-uniform particle size, resulting in suitable textures with good slipperiness and little roughness and squeakiness.

The unmodified cellulose beads have excellent soft-focus properties. Soft-focus properties require a high degree of light diffusion. It is known that the smaller the particle size of cellulose beads is, the higher the haze value is, and the greater the light diffusion effect tends to be. As described above, since the unmodified cellulose beads of the present invention contain a large number of fine particles, the light diffusion effect is enhanced and excellent soft-focus properties are obtained. Specifically, the haze value of a dispersion in which 0.05% by weight of the unmodified cellulose beads is dispersed in 50% trimethylsiloxysilicate and 50% cyclopentasiloxane is 50% or more, and more preferably 60% or more.

Thus, the unmodified cellulose beads of the present invention can achieve both textures and soft-focus properties.

Furthermore, the unmodified cellulose beads have a higher purity than conventional cellulose beads. Whiteness is used as an index of purity of cellulose beads. Conventional cellulose beads are produced from viscose obtained by, for example, chemically modifying cellulose. The viscose contains sulfur compounds such as by-products (sodium trithiocarbonate, etc.) and is colored yellow, and the cellulose after production also has residual sulfur compounds (sodium trithiocarbonate, etc.) and is yellowish, which reduces the whiteness of the powder. Conversely, since chemicals are not used in the unmodified cellulose beads of the present invention (not chemically modified), there is no influence of by-products, etc., in the manner of the prior art, and high whiteness, i.e., high purity, cellulose beads can be obtained. The preferable purity of the unmodified cellulose beads is a Hunter Whiteness (W value) of 85 or more and a Hunter Yellowness (YI value) of 20 or less.

EXAMPLES

[Preparation of Unmodified Cellulose Beads]

The unmodified cellulose beads of Trial Examples 1 to 7 were produced under the following conditions in accordance with the operation flowchart of FIG. 1. In Comparative Example 1, commercially available cellulose beads (CELLOBEADS D-10, manufactured by Daito Kasei Kogyo Co., Ltd.) were used.

<Trial Example 1>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. The sample was spray-dried using a spray dryer (manufactured by Ohkawara Kakohki Co., Ltd.) at an air pressure of 0.25 MPa (throughput of 75 kg/hr) (drying step). The particles were collected using a cyclone dust collector (manufactured by Ohkawara Kakohki Co., Ltd.) and then recovered from a bag filter (manufactured by Ohkawara Kakohki Co., Ltd.) (collection step), whereby the unmodified cellulose beads of Trial Example 1 were obtained.

<Trial Example 2>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. The sample was spray-dried using a spray dryer (manufactured by Ohkawara Kakohki Co., Ltd.) at an air pressure of 0.25 MPa (throughput of 75 kg/hr) (drying step). The particles were recovered by single-point collection using a bag filter (manufactured by Ohkawara Kakohki Co., Ltd.) (collection step), whereby the unmodified cellulose beads of Trial Example 2 were obtained.

<Trial Example 3>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. The sample was spray-dried using a spray dryer (manufactured by Ohkawara Kakohki Co., Ltd.) at an air pressure of 0.25 MPa (throughput of 75 kg/hr) (drying step). The particles were recovered using a cyclone dust collector (manufactured by Ohkawara Kakohki Co., Ltd.) (collection step), whereby the unmodified cellulose beads of Trial Example 3 were obtained.

<Trial Example 4>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. This sample was spray-dried using a spray dryer (manufactured by GF Corporation) at an air pressure of 0.6 MPa (throughput of 2.64 kg/hr) (drying step). The particles were recovered using a bag filter (manufactured by GF Corporation) (collection step), whereby the unmodified cellulose beads of Trial Example 4 were obtained.

<Trial Example 5>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. This sample was spray-dried using a spray dryer (manufactured by GF Corporation) at an air pressure of 0.06 MPa (throughput of 2.40 kg/hr) (drying step). The particles were recovered using a bag filter (manufactured by GF Corporation) (collection step), whereby the unmodified cellulose beads of Trial Example 5 were obtained.

<Trial Example 6>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. This sample was spray-dried using a spray dryer (manufactured by GF Corporation) at an air pressure of 0.025 MPa (throughput of 2.40 kg/hr) (drying step). The particles were recovered using a bag filter (manufactured by GF Corporation) (collection step), whereby the unmodified cellulose beads of Trial Example 6 were obtained.

<Trial Example 7>

18% by weight caustic soda was heated to 50°C, pulp was added so as to achieve 2% by weight, the mixture was stirred until it became a slurry, and mercerization was carried out (mercerization step). Excess caustic soda was then removed to adjust the solid content to 33% by weight. Aging treatment was performed at 50°C to reduce the degree of polymerization of the mercerized cellulose to 760 or less, whereby raw cellulose was obtained (depolymerization step). Ion-exchanged water and caustic soda were added to the treated sample, and defibration was performed using a homogenizer (manufactured by SMT Co., Ltd.) (defibration step). 96.0 kg of the prepared slurry was sampled and neutralized by adding 20% by weight sulfuric acid while stirring (neutralization step). The neutralized sample was dehydrated at 15 Hz in a centrifugal washer (manufactured by Matsumoto Machine Manufacturing Co., Ltd.) and washed for 30 minutes by spraying 5 L/min of ion-exchanged water. Ion-exchanged water was added to the washed sample so as to achieve a total weight of 96.0 kg, and the sample was then defibrated again using a homogenizer (manufactured by SMT Co., Ltd.) to obtain a dispersion of type II unmodified cellulose fine fibers. This sample was spray-dried using a spray dryer (manufactured by GF Corporation) at an air pressure of 0.02 MPa (throughput of 2.64 kg/hr) (drying step). The particles were recovered using a bag filter (manufactured by GF Corporation) (collection step), whereby the unmodified cellulose beads of Trial Example 7 were obtained.

To evaluate the performance of the unmodified cellulose beads of each of Trial Examples 1 to 7 and Comparative Example 1, the particle size distribution, haze (%), and whiteness were measured, and a sensory test of the textures was carried out.

[Particle Size Distribution]

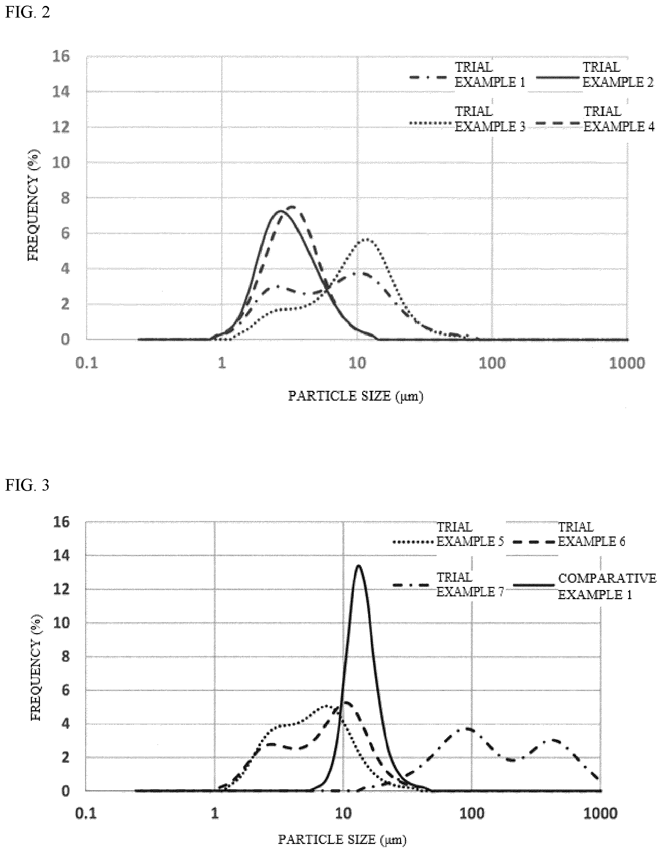

The particle sizes ($\mu$m) of the cellulose beads of Trial Examples 1 to 7 and Comparative Example 1 were measured in accordance with JIS Z8825 (2013). FIGS. 2 and 3 are graphs of the particle size distributions of the unmodified cellulose beads of Trial Examples 1 to 7 and Comparative Example 1 obtained by this measurement. A laser diffraction/scattering particle size distribution measuring device ("MT3200II" manufactured by Microtrack Bell Corp.) was used to measure the average particle size of each of the cellulose beads. In the measurement, a sample circulator was first filled with a slurry in which the cellulose beads were mixed with ion-exchanged water. This slurry was in a state in which it was stirred to the extent that no unevenness was visible to the naked eye immediately before being supplied. Thereafter, the sample was supplied to the measuring device, and each measurement parameter was set to a refractive index of 1.33 for ion-exchanged water, light transmittance of the particles to be measured treated as transparent, and a measurement time of 10 seconds. The particle size (D10) corresponding to 10% of the cumulative particle size distribution (volume basis), the particle size (D20) corresponding to 20% of the cumulative particle size distribution (volume basis), the particle size (D50) corresponding to 50% of the cumulative particle size distribution (volume basis), the particle size (D90) corresponding to 90% of the cumulative particle size distribution (volume basis), and the standard deviation (SD) of average particle size were measured.

[Haze]

A dispersion of 0.05% by mass of each of the cellulose beads of Trial Examples 1 to 7 and Comparative Example 1 was prepared, and the hazes (%) thereof were measured in accordance with JIS K7136 (2000). Haze (%) is an index of transparency, and a haze meter ("NDH-4000" manufactured by Nippon Denshoku Industries Co., Ltd.) was used. The dispersion was placed in a glass cell for liquids (manufactured by Fujiwara Seisakusho Co., Ltd.: "MG-40") having an optical path of 1 cm using a 50% trimethylsiloxysilicate/50% cyclopentasiloxane solution (manufactured by Shin-Etsu Chemical Co., Ltd.: "KF7312J") to adjust the concentration. Zero-point measurement was performed by putting a 50% trimethylsiloxysilicate/50% cyclopentasiloxane solution into the same glass cell. The hazes of the cellulose beads of Trial Examples 1 to 7 and Comparative Example 1 were evaluated as "Good (G)" when the measurement result was 50% or more, and "Poor (P)" when the measurement result was less than 50%.

[Whiteness]

The Hunter Whiteness (W value) and Hunter Yellowness (YI value) of the cellulose beads of Trial Examples 1 to 7 and Comparative Example 1 were calculated in accordance with JIS P8123. Whiteness is an index of cellulose purity, and a spectrophotometer ("SE7700" manufactured by Nippon Denshoku Industries Co., Ltd.) was used to determine the chromaticity coordinates X, Y, and Z defined by the CIE (Commission Internationale de l'Eclairage) by comparison with a standard white plate (X=91.2, Y=96.1, Z=104.5), and whiteness (W value) and yellowness (YI value) were calculated using the following formula:

$$\text{Whiteness (W value)} = Z/1.18$$

$$\text{Yellowness (YI value)} = (X - 0.98 \times W / 0.8) \times Y$$

The whiteness of the cellulose beads of Trial Examples 1 to 7 and Comparative Example 1 was evaluated such that a Hunter Whiteness (W value) of 85 or more and a Hunter Yellowness (YI value) of 20 or less was evaluated as "Good (G)"; and a Hunter Whiteness (W value) of less than 85 or a Hunter Yellowness (YI value) of greater than 20 was evaluated as "Poor (P)".

[Sensory Test]

The cellulose beads of Trial Examples 1 to 7 and Comparative Example 1 were spread on the skin with the fingers, and ten testers evaluated the slipperiness, roughness, and squeakiness on a five-point scale for each. The texture of each item was evaluated such that an average score of 5.0 to 4.5 was evaluated as "E", 4.4 to 3.5 was evaluated as "G", 3.5 to 2.5 was evaluated as "OK", and 2.4 or less was evaluated as "X".

[Comprehensive Evaluation]

The measurement results and test results of the cellulose beads of Trial Examples 1 to 7 and Trial Example 1 and their ratings are shown in Tables 1 and 2. For the comprehensive evaluation, two or more "OK" or at least one "X" was evaluated as "Poor (P)", no "X" but at least one "OK" was evaluated as "OK", and ratings above that were evaluated as "Good (G)".

[Table 1]

| | | Trial Ex 1 | Trial Ex 2 | Trial Ex 3 | Trial Ex 4 |
|---|---|---|---|---|---|
| D50 ($\mu$m) | | 6.60 | 2.90 | 9.34 | 3.14 |
| D10 ($\mu$m) | | 1.92 | 1.67 | 2.72 | 1.73 |
| D20 ($\mu$m) | | 2.58 | 2.00 | 4.41 | 2.13 |
| D90 ($\mu$m) | | 19.27 | 5.71 | 19.53 | 5.74 |
| SD/D50 (CV value) | | 0.99 | 0.52 | 0.69 | 0.48 |
| Air pressure (mPa) | | 0.250 | 0.250 | 0.250 | 0.600 |
| Haze | Measured value (%) | 80.95 | 97.18 | 65.72 | 96.91 |
| | Judgment | G | G | G | G |
| Whiteness | W value | 94.84 | 96.41 | 93.54 | 97.17 |
| | YI value | 3.43 | 3.41 | 3.61 | 2.00 |
| | Judgment | G | G | G | G |
| Slipperiness | | G | G | E | G |
| Roughness | | G | G | G | G |
| Squeakiness | | G | G | G | G |
| Comprehensive evaluation | | G | G | G | G |

[Table 2]

|  |  | Trial Ex 5 | Trial Ex 6 | Trial Ex 7 | Comp Ex 1 |
|---|---|---|---|---|---|
| D50 ($\mu$m) | | 5.48 | 7.32 | 133 | 13.10 |
| D10 ($\mu$m) | | 2.25 | 2.17 | 43.55 | 9.54 |
| D20 ($\mu$m) | | 2.89 | 2.99 | 61.83 | 10.63 |
| D90 ($\mu$m) | | 12.11 | 15.81 | 561.6 | 19.31 |
| SD/D50 (CV value) | | 0.7 | 0.74 | 1.53 | 0.28 |
| Air pressure (mPa) | | 0.060 | 0.025 | 0.020 | - |
| Haze | Measured value (%) | 86.28 | 79.58 | 11.53 | 41.60 |
| | Judgment | G | G | P | P |
| Whiteness | W value | 93.92 | 92.57 | 90.02 | 84.09 |
| | YI value | 5.32 | 2.75 | 6.36 | 20.52 |
| | Judgment | G | G | G | P |
| Slipperiness | | G | G | P | G |
| Roughness | | G | E | P | G |
| Squeakiness | | G | G | G | P |
| Comprehensive evaluation | | G | G | P | P |

[Results and Discussion]

The commercially available cellulose beads of Comparative Example 1 have a particle size distribution that is sharp at a particle size of approximately 10 $\mu$m, as shown in FIG. 3, and have a CV value (SD/D50) of 0.28. As can be seen therefrom, the particle size of the cellulose beads of Comparative Example 1 is relatively uniform at approximately 10 $\mu$m. As a result, while the textures in terms of slipperiness and roughness were suitable, there was strong squeakiness due to the uniformity of the particle size, and in addition, the haze value was low, which indicated that the soft-focus properties were insufficient.

Conversely, as shown in FIGS. 2 and 3, the cellulose beads of Trial Examples 1 to 7 have a relatively flat particle size distribution (horizontally spread graph) and a high CV value (SD/D50) as compared to Comparative Example 1, indicating that the particle size varies widely, and specifically, the particle size is relatively non-uniform. The cellulose beads of Trial Examples 1 to 7 exhibited an improvement in squeakiness as compared to the cellulose beads of Comparative Example 1.

In the cellulose beads of Trial Examples 1 to 6, in particular, in Trial Examples 1 to 7, the textures in terms of slipperiness and roughness as well as squeakiness were all suitable, and the haze was high, which indicated excellent soft-focus properties. In Trial Example 7, the textures in terms of slipperiness and roughness were poor, and the haze was low, which indicated the soft-focus properties were lacking.

When Trial Examples 1 to 6 and Comparative Example 1 are compared with each other, it is shown that the cellulose beads of Trial Examples 1 to 6 had a particle size distribution flatter on the smaller particle size side as compared to the cellulose beads of Comparative Example 1, which indicates that the particles as a whole in Trial Examples 1 to 6 were relatively non-uniform in particle size and a large number of particles (fine particles) having smaller particle sizes were contained as compared to Comparative Example 1. Though it has conventionally been considered good that a particle size be relatively uniform (a sharp peak appears in the particle size distribution), Trial Examples 1 to 6 had an overall non-uniform particle size and contained a large number of fine particles (a slightly flat peak appears on the smaller diameter side in the particle size distribution), which made it possible to achieve both textures and soft-focus properties.

It is shown that the cellulose beads of Trial Example 7 contained a large number of particles having a large particle size as compared to Comparative Example 1, with the overall particle size being relatively non-uniform. Though the cellulose beads of Trial Example 7 had improved squeakiness as compared to Comparative Example 1, the other textures, slipperiness and roughness, were reduced, and haze was low, which indicated reduced soft-focus properties. From the differences between the characteristics of Trial Examples 1 to 6 and the characteristics of Trial Example 7 relative to Comparative Example 1, it is clear that in order to achieve both textures and soft-focus properties, it is preferable for the particle diameter of the cellulose beads to be non-uniform and for a large number of fine particles to be contained in the cellulose beads.

The cellulose beads of Comparative Example 1 were chemically modified cellulose beads, and the Hunter Whiteness (W value) and Hunter Yellowness (YI value) thereof indicated a tendency to be weakly white and strongly yellow. Conversely, the cellulose beads of Trial Examples 1 to 7 were unmodified cellulose beads, and all had suitable Hunter Whiteness (W value) and Hunter Yellowness (YI value), indicating that they were of higher purity than Comparative Example 1.

INDUSTRIAL APPLICABILITY

According to the unmodified cellulose beads and production method therefor of the present invention, both textures and soft-focus properties suitable for cosmetics can be achieved. Thus, they offer promise as an alternative to microplastics and chemically modified cellulose beads which are used in conventional cosmetics.

DESCRIPTION OF REFERENCE SIGNS

S1    mercerization step
S2    depolymerization step
S3    defibration step
S4    neutralization step
S5    drying step
S6    collection step

**Claims**

1.    Unmodified cellulose beads for mixing into a cosmetic, having:

an average particle diameter (D50) of 1 to 20 $\mu$m,
an average particle diameter (D10) of 4.0 $\mu$m or less,
an average particle diameter (D20) of 6.0 $\mu$m or less, and
an average particle diameter (D90) of 30.0 $\mu$m or less, wherein
a standard deviation (SD) of average particle size divided by the average particle diameter (D50) (SD/D50) is 0.4 to 1.5.

2.    The unmodified cellulose beads according to claim 1, which are produced from a raw material pulp without being subjected to a defibration step by chemical defibration.

3.    The unmodified cellulose beads according to claim 2, having a Hunter Whiteness (W value) of 85 or more and a Hunter Yellowness (YI value) of 20 or less.

4.    The unmodified cellulose beads according to claim 1, wherein a dispersion in which 0.05% by weight of the unmodified cellulose beads are dispersed in 50% trimethylsiloxysilicate and 50% cyclopentasiloxane has a haze value of 50% or more.

5.    A production method for the unmodified cellulose beads according to claim 1, the method comprising:

a mercerization step of mercerizing a cellulose to obtain mercerized cellulose,
a depolymerization step for reducing a degree of polymerization of the mercerized cellulose to 760 or less,
a defibration step in which a raw cellulose obtained via the mercerization step and the depolymerization step is defibrated by adding an alkali metal hydroxide to the raw cellulose so that the total concentration of the alkali metal hydroxide is 2.5 to 17.5%, to obtain cellulose fine fibers,
a neutralization step of neutralizing the cellulose fine fibers with an acid, and
a drying step of spray-drying the neutralized cellulose fine fibers at an air pressure of 0.025 to 0.6 MPa.

FIG. 1

MERCERIZATION STEP (S1)
- CELLULOSE
- ALKALI METAL HYDROXIDE
- HEATING/ STIRRING

DEPOLYMERIZATION STEP (S2)
- MERCERIZED CELLULOSE
- AGING

DEFIBRATION STEP (S3)
- RAW CELLULOSE
- ALKALI METAL HYDROXIDE
- SOLVENT
- DEFIBRATION

NEUTRALIZATION STEP (S4)
- CELLULOSE FINE FIBERS
- ACID
- NEUTRALIZATION/WASHING /RE-DEFIBRATION

DRYING STEP (S5)
- TYPE II UNMODIFIED CELLULOSE FINE FIBERS
- SPRAY-DRYING

COLLECTION STEP (S6)
- COLLECTION
- UNMODIFIED CELLULOSE BEADS

FIG. 2

FIG. 3

## INTERNATIONAL SEARCH REPORT

| International application No. |
|---|
| **PCT/JP2023/019567** |

### A. CLASSIFICATION OF SUBJECT MATTER

*C08B 1/00*(2006.01)i; *C08J 3/12*(2006.01)i; *A61Q 19/00*(2006.01)i; *A61K 8/73*(2006.01)i
FI: C08B1/00; C08J3/12 101; A61K8/73; A61Q19/00; C08J3/12 CEP

According to International Patent Classification (IPC) or to both national classification and IPC

### B. FIELDS SEARCHED

Minimum documentation searched (classification system followed by classification symbols)

C08B1/00; C08J3/12; A61Q19/00; A61K8/73

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2021/209075 A1 (MUDANJIANG LINRUN PHARMA EXCIPIENTS LLC.) 21 October 2021 (2021-10-21)<br>claims, examples | 1-4 |
| A | JP 2019-206662 A (TORAY INDUSTRIES) 05 December 2019 (2019-12-05)<br>entire text | 1-5 |
| A | WO 2019/111933 A1 (JUJO PAPER CO LTD) 13 June 2019 (2019-06-13)<br>entire text | 1-5 |
| A | JP 2004-536155 A (BKI HOLDING CORPORATION) 02 December 2004 (2004-12-02)<br>entire text | 1-5 |
| A | JP 2015-157796 A (SUGINO MACH) 03 September 2015 (2015-09-03)<br>entire text | 1-5 |
| P, A | WO 2023/017687 A1 (FUTAMURA KAGAKU KK) 16 February 2023 (2023-02-16)<br>entire text | 1-5 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 July 2023** | **08 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 549 471 A1

**INTERNATIONAL SEARCH REPORT**
**Information on patent family members**

International application No.

**PCT/JP2023/019567**

| Patent document cited in search report | | | Publication date (day/month/year) | Patent family member(s) | | | Publication date (day/month/year) |
|---|---|---|---|---|---|---|---|
| WO | 2021/209075 | A1 | 21 October 2021 | EP | 4137531 | A1 | |
| | | | | claims, examples | | | |
| | | | | CN | 111333875 | A | |
| JP | 2019-206662 | A | 05 December 2019 | (Family: none) | | | |
| WO | 2019/111933 | A1 | 13 June 2019 | US | 2021/0214465 | A1 | |
| | | | | EP | 3722325 | A1 | |
| | | | | CN | 111433225 | A | |
| | | | | KR | 10-2020-0092398 | A | |
| JP | 2004-536155 | A | 02 December 2004 | US | 2002/0103368 | A1 | |
| | | | | WO | 2002/036636 | A1 | |
| | | | | EP | 1334132 | A1 | |
| | | | | CN | 1503807 | A | |
| JP | 2015-157796 | A | 03 September 2015 | (Family: none) | | | |
| WO | 2023/017687 | A1 | 16 February 2023 | JP | 2023-25376 | A | |

Form PCT/ISA/210 (patent family annex) (January 2015)

16

**EP 4 549 471 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008001728 A
- JP 2019099758 A
- WO 2019059079 A